# EUROPÄISCHE PATENTANMELDUNG

(11) **EP 4 509 078 A1**
(43) Veröffentlichungstag der Anmeldung: **19.02.2025**
(21) Anmeldenummer: 23191383.1
(22) Anmeldetag: 14.08.2023
(51) Int. Cl.: A61B 18/14, A61M 25/00

(54) **ABLATIONSINSTRUMENT UND HERSTELLUNGSVERFAHREN FÜR DIESES**

(71) Anmelder: Erbe Elektromedizin GmbH, 72072 Tübingen (DE)
(72) Erfinder: Adler, Marcus, 72393 Burladingen (DE); Karcher, Felix, 72072 Tuebingen (DE); Stäbler, Holger, 71093 Weil im Schoenbuch (DE)
(74) Vertreter: Rüger Abel Patentanwälte PartGmbB

(57) **Zusammenfassung**

Das erfindungsgemäße innen gekühlte Ablationsinstrument (11) weist im Anschluss an sein distales Ende (12) einen Krümmungsbereich (20) auf, bei welchem das Ablationsinstrument (11) gegen seine Axialrichtung (A) abgewinkelt ist. Die Größe dieses Winkels ist vorzugsweise auf ein Drittel bis zwei Drittel vorzugsweise die Hälfte der maximalen Abwinklung festgelegt. Die maximale Abwinklung kann einen Betrag von 140° deutlich überschreiten. Durch die Vorkrümmung in dem Krümmungsbereich 20 wird ein Abknicken des Schlauchs des Ablationsinstruments und somit eine Beeinträchtigung der Elektrodenkühlung oder den Aufbau eines hohen Staudruckes wirksam verhindert. Damit gestattet das erfindungsgemäße Ablationsinstrument (11) eine im Vergleich zu vollkommen gestreckten Ablationsinstrumenten freieres und noch stärker an medizinischen Aspekten und Gesichtspunkten ausgerichtetes Arbeiten. Die Behandlungssicherheit für den Patienten wird deutlich erhöht.

## Beschreibung

Die Erfindung betrifft ein Ablationsinstrument, insbesondere zur thermischen Ablation eines Gewebevolumens durch Bestromung, und im Weiteren ein Verfahren zur Herstellung eines solchen Ablationsinstruments.

Die EP 3 788 974 A1 offenbart ein Ablationsinstrument mit schlauchartigem Grundkörper, an dessen distalem Ende zwei Ablationselektroden angeordnet sind. Der Schlauch ist flexibel, sodass sich die Sonde, wenn sie in den Arbeitskanal eines Endoskops eingesetzt ist, in verschiedenen Richtungen abwinkeln lässt.

Weiter offenbart die US 4,474,174 A ein chirurgisches Instrument zur Verwendung in einem flexiblen Endoskop. Dieses Instrument umfasst einen Katheter, in welchem ein chirurgisches Werkzeug angeordnet ist. Dieser Katheter weist eine vorgegebene Biegung und in dem Bereich der Biegung eine Öffnung auf, aus der ein in dem Katheter geführtes Werkzeug austreten kann.

Die US 6,017,340 A offenbart ein weiteres Instrument zur endoskopischen Behandlung, insbesondere des Gallengangs. Dieses Instrument weist ein distales bogenförmig gekrümmtes Ende auf, das von einer drahtförmigen Elektrode überspannt wird.

Zur Durchführung von Eingriffen an schwer zugänglichen Körperstellen offenbart die US 2,022,065 A ein Instrument mit spitzwinklig zurückgebogenem distalem Ende.

Die EP 0 536 440 B1 beschreibt ein am distalen Ende gekrümmtes HF-chirurgisches Instrument für den endoskopischen und/oder laparoskopischen Einsatz im Rektum. Die vorgegebene Biegung des Instruments erleichtert die Führung des Instruments in einem Kanal eines Endoskops, dessen distales Ende eine feste abgewinkelte Form aufweist.

Weiterer Stand der Technik wird durch die EP 0 947 171 B1 und die EP 1 076 523 A1 gebildet.

Für den korrekten Betrieb einer Ablationssonde ist wesentlich, dass die mit dem Gewebe in Kontakt stehende Elektrode nicht zu warm wird, um ein Austrocknen des mit der Elektrode in Kontakt stehenden Gewebes zu verhindern. Um dies zu erreichen, kann das innere Lumen des schlauchförmigen Instruments als Kühlkanal genutzt werden. Dieser Kühlkanal muss auch dann fluiddurchlässig sein, wenn die Sonde vom Endoskop in eine abgewinkelte Form überführt wird.

Es ist Aufgabe der Erfindung, ein Ablationsinstrument zu schaffen, das sich beim endoskopischen Einsatz ohne Einschränkung seiner Funktionsfähigkeit in abgewinkelte Form überführen lässt. Dabei sollen insbesondere große Angulationen und enge Biegeradien ermöglicht werden. Außerdem ist es Aufgabe der Erfindung, ein Verfahren zur Herstellung eines solchen Ablationsinstruments anzugeben.

Diese Aufgaben werden gemäß den Ansprüchen 1 und 15 gelöst:
Das erfindungsgemäße Ablationsinstrument weist als Grundkörper einen flexiblen Schlauch mit wenigstens einem Lumen auf, das sich von seinem proximalen Ende bis zu seinem distalen Ende erstreckt. Dieses Lumen ist als Kühlkanal nutzbar, um die wenigstens eine an dem distalen Ende des Schlauchs angeordnete Elektrode vor übermäßiger Erwärmung zu bewahren, d.h. sie zu kühlen. Die Elektrode kann dabei in einem ohne Einwirkung äußerer Kräfte nicht gekrümmten Abschnitt des Schlauchs angeordnet sein. In Nachbarschaft der Elektrode weist der Schlauch einen Krümmungsbereich auf, in dem er ohne auf das Ablationsinstrument einwirkende äußere Kräfte einem Bogen folgend ausgebildet ist. Sowohl der die Elektrode tragende vorzugsweise gerade ausgebildete distale Endbereich des Schlauchs als auch der dem Bogen folgende Bereich, wie auch der sich in proximaler Richtung daran anschließende wiederum vorzugsweise gerade Bereich sind flexibel ausgebildet. Damit können die im Ruhezustand ungekrümmten Bereiche bedarfsweise gekrümmt werden. Umgekehrt kann der gekrümmte Bereich weiter gekrümmt oder auch in eine gerade gestreckte Form überführt werden. Damit lässt sich das Ablationsinstrument, obwohl es einen Krümmungsbereich aufweist, in den geraden Arbeitskanal eines Endoskops einschieben oder aus diesem herausziehen. Bei diesem Vorgang wird der Krümmungsbereich in eine ungekrümmte Form überführt.

Der Krümmungsbereich kann eine Steifigkeit aufweisen, die größer ist als die Steifigkeit des sich in proximaler Richtung an den Krümmungsbereich anschließenden Abschnitts des Ablationsinstruments. Auch kann die Steifigkeit des Krümmungsabschnitts größer sein als die Steifigkeit des sich in distaler Richtung an den Krümmungsbereich anschließenden Abschnitts des Ablationsinstruments. Alternativ kann jedoch die Steifigkeit des distalen Abschnitts des Ablationsinstruments auch größer sein als die des Krümmungsbereichs. Dadurch kann das Einstechen des Ablationsinstruments in festes Gewebe (z.B. einen soliden Tumor) erleichtert werden.

Der Krümmungsbereich ist vorzugsweise in demjenigen Bereich des Schlauchs vorgesehen, der sich in dem Abwinkelbereich eines Endoskops befindet, wenn die Sonde mit ihrem distalen Ende so aus dem Arbeitskanal eines Endoskops herausgeschoben ist, dass ihre Elektrode oder ihre Elektroden in das zu behandelnde Gewebe eingestochen sind. Das Ablationsinstrument kann bedarfsweise auch mehrere Krümmungsbereiche aufweisen.

Der Krümmungsbereich legt vorzugsweise einen Krümmungswinkel fest, der ungefähr der Hälfte der von dem Ablationsinstrument zu absolvierenden Abwinklung entspricht. Werden beispielsweise Angulationen (d.h. Abwinkelungen) von größer gleich 145° (zum Beispiel 160°) gewünscht, legt der Krümmungsbereich vorzugsweise einen Winkel zur Längsrichtung von 30° bis 70° fest. Je nach Anforderung sind auch andere Winkel möglich. Das Ablationsinstrument kann einerseits durch elastische Verformung ohne Schaden zu nehmen in eine gestreckte Form überführt werden, um durch den Arbeitskanal eines Endoskops bis in das Gewebe des Patienten eingeschoben zu werden. Andererseits kann der Krümmungsbereich des Ablationsinstruments durch entsprechende Abwinklung des distalen Endes des Endoskops bis zu einem Angulationswinkel von 140° bis 160° gekrümmt werden, wobei die Krümmung des Schlauchs im Krümmungsbereich zunimmt, jedoch ohne Gefahr, dass der Schlauch abgeknickt wird und somit sein Lumen kollabiert.

Das Lumen des Schlauchs ist vorzugsweise an dem distalen Ende geschlossen. Zum Verschluss des Schlauchs kann ein aushärtbarer Kunststoff dienen, der einen das Schlauchende verschließenden Pfropfen bildet. Vorzugsweise ist in das Schlauchende jedoch ein Verschlussstück eingesetzt, das wenigstens teilweise aus einem elektrisch leitfähigen Material, beispielsweise Metall besteht. Wenn das Verschlussstück mit einem eigenen elektrischen Anschluss versehen ist, der sich beispielsweise durch das Lumen des Schlauchs erstrecken kann, kann es als Elektrode genutzt werden, um das Einstechen des Instruments in biologisches Gewebe zu erleichtern und/oder Blutungen zu stillen (Koagulation). Außerdem kann das Verschlussstück mit einem sich von dem distalen Ende zu dem proximalen Ende des Schlauchs erstreckenden Zugmittel verbunden sein, um eine Schädigung des Instruments beim Herausziehen aus koaguliertem Gewebe zu vermeiden.

In dem Schlauch kann ein Kapillarkanal vorgesehen sein, um ein Kältemittel zu dem distalen Ende des Ablationsinstruments zu führen, um von der Elektrode ausgehende Wärme aufzunehmen und abzuleiten. Der Kapillarkanal kann in dem Schlauch selbst, zum Beispiel als zweites Lumen ausgebildet sein. Alternativ kann der Kapillarkanal in einem Kapillarrohr ausgebildet sein, das in dem Lumen angeordnet ist und sich von dem proximalen Ende bis zu dem distalen Ende erstreckt. Der Kapillarkanal ist mit einer oder mehreren Öffnungen versehen, aus denen in der Nähe der Elektrode ein Kältemittel in das Lumen des Schlauchs übertreten kann.

Die Elektrode ist vorzugsweise den Schlauch umfassend und dabei so ausgebildet, dass sie in allen Radialrichtungen gleichwirkend ausgebildet ist. Die Elektrode kann beispielsweise als Zylinderelektrode ausgebildet oder durch einen den Schlauch schraubenförmig umwindenden elektrischen Leiter gebildet sein. Damit spielt die axiale Orientierung des Ablationsinstruments für die Wirkung auf das Gewebe keine Rolle. Das Ablationsinstrument kann deswegen, wenn es in ein gestrecktes gerades Endoskop eingeführt wird, welches danach abgewinkelt wird, durch eine Drehung um seine Axiale diejenige Drehposition einnehmen, in der die Krümmung des Ablationsinstruments in die gleiche Richtung geht, wie die Krümmung des Endoskops.

An dem distalen Ende des Schlauchs kann auf dem nicht gekrümmten Bereich wenigstens eine weitere Elektrode angeordnet sein. Der distal zu dem Krümmungsbereich gelegene distale Endbereich des Schlauchs trägt dann mindestens zwei Elektroden, die beispielsweise zur Gewebeablation, insbesondere zur Tumorablation, genutzt werden können, indem sie mit zwei Polen eines Generators verbunden werden. Dieser ist vorzugsweise darauf eingerichtet, eine elektrische hochfrequente Spannung zu erzeugen, um einen elektrischen Strom durch das mit den beiden Elektroden in Kontakt stehende Gewebe fließen zu lassen, um das Gewebe zu koagulieren. Prinzipiell ist jedoch auch die Nutzung lediglich einer Elektrode an dem Ablationsinstrument möglich, wobei dann als Gegenelektrode eine Neutralelektrode am Patienten zu befestigen ist. Die Ausführungsform mit zwei Elektroden hat jedoch Vorteile, insbesondere beim Einsatz in einer hochohmigen Umgebung, beispielsweise bei der Ablation solider Tumore in Lungengewebe. Die Elektrode oder die Elektroden können auch ganz oder teilweise in dem gekrümmten Bereich des Instruments angeordnet sein.

Bei dem erfindungsgemäßen Ablationsinstrument ist auf dem Schlauch ein Mantel angeordnet, der mit dem Schlauch einen Spalt bzw. spaltartigen Zwischenraum bildet. Der Mantel kann sich insbesondere von dem proximalen Ende des Ablationsinstruments bis durch den Krümmungsbereich hindurch zu der Elektrode oder bis zu einem an die Elektrode anschließenden Isolator erstrecken. Der zwischen dem Schlauch und dem Mantel ausgebildete Spalt ermöglicht eine axiale Relativbeweglichkeit zwischen dem Mantel und dem Spalt, was der Flexibilität des Ablationsinstruments zugutekommt.

In dem Krümmungsbereich kann der Spalt mit einem Kunststoff, insbesondere einem 2-Komponenten-Kunststoff, gefüllt sein, um dort eine feste Verbindung zwischen dem Mantel und dem Schlauch zu schaffen. Dieser Kunststoff kann dazu benutzt werden, die Krümmung des Ablationsinstruments in dem Krümmungsbereich festzulegen. Vorzugsweise ist der Kunststoff ein flexibler Kunststoff, sodass die Krümmung durch Einwirkung äußerer Kräfte beseitigt oder auch vergrö-ßert werden kann. Der Kunststoff ist vorzugsweise ein aushärtbarer Kunststoff, der bei der Herstellung des Ablationsinstruments in den Spalt eingebracht wird, wobei oder wonach der Krümmungsbereich in seine gekrümmte Form überführt und der Kunststoff ausgehärtet wird. Dadurch weist das Ablationsinstrument nach dem Aushärten des Kunststoffs einen Bereich mit permanenter Krümmung auf. Dies obwohl sowohl der Schlauch als auch der Mantel zwar flexibel, jedoch in unbeeinflusstem Zustand gerade, d.h. ungekrümmt ausgebildet sind. Die Kunststofffüllung des Spalts bewirkt eine Aussteifung des Querschnitts, so dass das Lumen in dem mit der Kunststofffüllung versehenen Bereich eine verminderte Empfindlichkeit gegen Kollaps infolge Abknickens des Ablationsinstruments aufweist.

Einzelheiten von Ausführungsformen der Erfindung sowie gegebenenfalls auch weitere Aspekte derselben ergeben sich aus der Zeichnung, der zugehörigen Beschreibung sowie Ansprüchen. Es zeigen:
Figur 1 ein in ein Endoskop eingesetztes Ablationsinstrument, angeschlossen an ein speisendes Gerät, in schematisierter Darstellung,
Figur 2 das erfindungsgemäße Ablationsinstrument, in einer ausschnittsweisen Darstellung,
Figur 3 das distale Ende des Ablationsinstruments nach Figur 2 in vergrößerter Seitenansicht,
Figur 4 das distale Ende des Ablationsinstruments gemäß Figur 3, jedoch längs geschnitten,
Figur 5 einen Ausschnitt aus dem Krümmungsbereich des Ablationsinstruments nach den Figuren 1 bis 4, in schematisierter längsgeschnittener Darstellung,
Figur 6 einen Ausschnitt aus dem Krümmungsbereich eines Ablationsinstruments nach den Figuren 1 bis 4, jedoch in abgewandelter Ausführungsform, in schematisierter längsgeschnittener Darstellung.

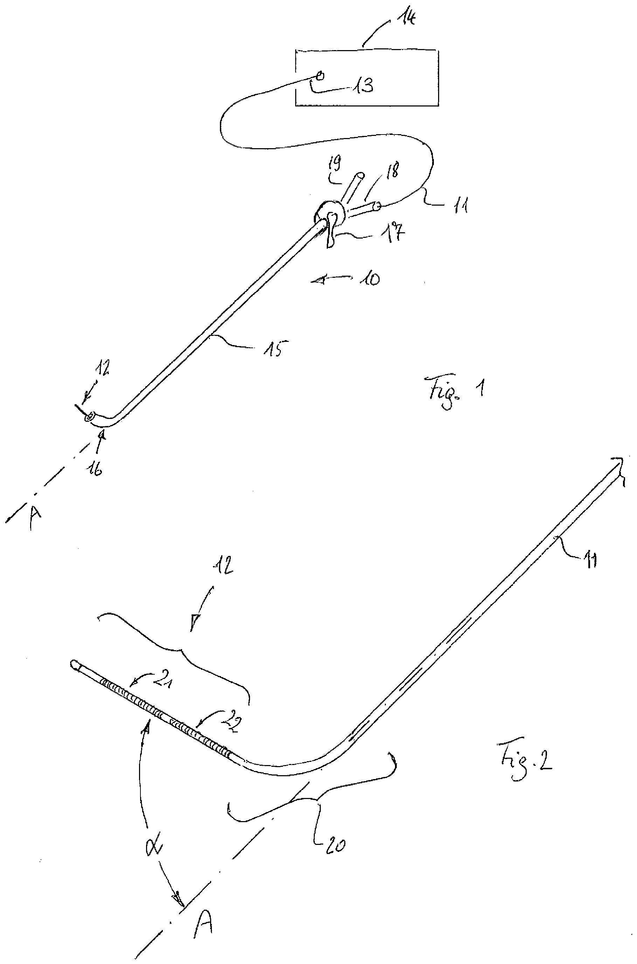

In Figur 1 ist eine in ein Endoskop 10 eingesetzte Ablationssonde 11 veranschaulicht, deren distales Ende 12 aus dem Arbeitskanal des Endoskops 10 herausragt. Das proximale Ende 13 der Ablationssonde 11 ist hingegen an ein speisendes Gerät 14 oder einen Geräteverbund angeschlossen, das die Ablationssonde 11 mit Strom, Spannung und Kühlmedium versorgt.

Das Endoskop 10 weist einen länglichen geraden, sich entlang einer Axialrichtung A erstreckenden Schaft 15 auf, dessen distales Ende 16 sich durch ein am proximalen Ende des Schafts 15 vorgesehenes Bedienelement 17, beispielsweise ein Stellrad, Stellhebel oder dergleichen, mehr oder weniger seitlich abwinkeln lässt. In der Nähe dieses Bedienelements 17 weist das Endoskop 10, je nach Anzahl der vorhandenen Arbeitskanäle, ein oder mehrere Anschlüsse 18, 19 auf, über die sich Sonden, Instrumente oder dergleichen, in den jeweiligen Arbeitskanal des Endoskops 10 einführen lassen.

Das Endoskop 10 ist vorzugsweise zum Arbeiten in engen und verwinkelten Lumen eines Körpers eines Patienten vorgesehen. Es kann z.B. zur Behandlung von Lungengewebe, insbesondere von Tumoren im Lungengewebe, eingesetzt werden. Das distale Ende 16 des Endoskops 10 kann dazu durch entsprechende Betätigung des Bedienelements 17 in eine gestreckte Form gebracht oder auch um einen Winkel von z.B. bis zu 140° oder mehr (150°, 160°) gegen die Axialrichtung A abgewinkelt werden. Dabei können Biegeradien erzeugt werden, die kleiner als 15 mm sind.

Figur 2 veranschaulicht die Ablationssonde 11 gesondert anhand deren distalen Endes 12 und des sich proximal daran anschließenden Abschnitts. Dieser Abschnitt enthält unmittelbar anschließend an das distale Ende 12 einen Krümmungsbereich 20, in welchem die Ablationssonde 11 ohne Einfluss äußerer Kräfte gekrümmt ist. Hingegen sind das distale Ende 12 sowie der von dem Krümmungsbereich 20 ausgehend sich bis zu dem proximalen Ende 13 der Sonde erstreckende Abschnitt ohne Einfluss äußerer Kräfte vorzugsweise gerade. Dieser gerade, von dem Krümmungsbereich 20 in proximaler Richtung verlaufende Abschnitt der Ablationssonde 11 legt eine Längsrichtung fest, die mit der Axialrichtung A des Arbeitskanals des Schafts 15 übereinstimmt, wenn das Ablationsinstrument 11 in den Arbeitskanal eingeschoben ist. Der von dem Krümmungsbereich 20 festgelegte Winkel α zwischen dem distalen Ende 12 und der Axialrichtung A ist vorzugsweise etwa halb so groß, wie die von dem distalen Ende 16 des Schafts 15 zu erzielende maximale Angulation. Soll eine maximale Angulation bzw. Abwinklung gegenüber der Axialrichtung A von zum Beispiel 150° erreicht werden, kann der Winkel α vorzugsweise zwischen 30° und 70°, vorzugsweise etwa 50° (+/- 20°) betragen. Somit ist die nötige Verformung des Ablationsinstruments 11 aus der Ruheform in die maximale gestreckte Form oder aus der Ruheform in die maximale abgewinkelte Form jeweils lediglich so gering, dass ein Abknicken der Ablationssonde an der Biegestelle nicht zu befürchten ist.

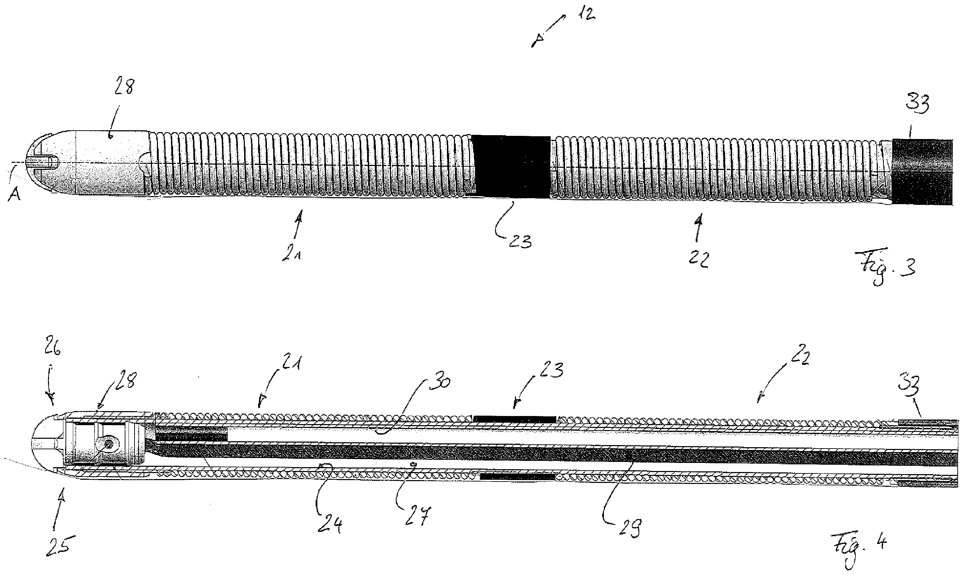

Die Ablationssonde 11 weist auf ihrem distalen Ende 12 wenigstens eine, vorzugsweise zwei Elektroden 21, 22 auf, die starr oder auch flexibel sein können. Die Elektroden 21, 22 können untereinander gleich ausgebildet sein. Sie weisen in jeder Radialrichtung gleiche elektrische Aktivität auf. Mit anderen Worten, sie erstrecken sich um die gesamte Umfangsrichtung der Ablationssonde 11 herum. Wie insbesondere aus Figur 3 hervorgeht, können die Elektroden 21, 22 durch einen schraubenförmig gewundenen elektrischen Leiter, beispielsweise einen Draht, gebildet sein. Zwischen den beiden Elektroden 21, 22 kann ein Isolator 23 vorgesehen sein.

Wie Figur 4 erkennen lässt, sind die beiden Elektroden 21, 22, wie vorzugsweise auch der hülsenförmige Isolator 23, auf einem Schlauch 24 angeordnet, der sich von dem proximalen Ende 13 über die gesamte Länge des Ablationsinstruments 11 durch die Elektroden 21, 22 hindurch bis zu der Instrumentenspitze 25 erstreckt. Die Instrumentenspitze 25 wird durch ein Endstück 26 gebildet, mit dem das von dem Schlauch 24 umschlossene Lumen 27 verschlossen wird. Das Endstück 26 kann zum Beispiel aus Metall bestehen und eine kreuzförmige Spitze aufweisen. Ein Isolatorelement 28 kann das Endstück 26 teilweise überdecken und sich über den Schlauch 24 erstrecken. Das Endstück 26 kann über ein Zugmittel 29, zum Beispiel in Gestalt eines Drahts oder dergleichen, mit dem proximalen Ende des Ablationsinstruments 11 oder einem Bereich des Instruments verbunden sein, der bei einer endoskopischen Anwendung des Ablationsinstruments außerhalb des Endoskops verbleibt. Außerdem kann in dem Lumen 27 eine Fluidleitung angeordnet sein, die von dem proximalen Ende des Ablationsinstruments 11 her ein Kühlmittel bis zu dem distalen Ende 12 führt, um damit die Elektroden 21, 22 zu kühlen. Die Fluidleitung kann insbesondere im distalen Bereich ein Kapillarrohr 30 aufweisen, das auch zur Zugkraftübertragung dienen kann. Das Kapillarrohr 30 kann eine oder mehrere Kühlmittelauslassöffnungen zum Beispiel an seinem distalen Ende oder in seiner Wandung im Bereich der Elektroden 21, 22 aufweisen, um dort eine Kühlwirkung zu erzielen.

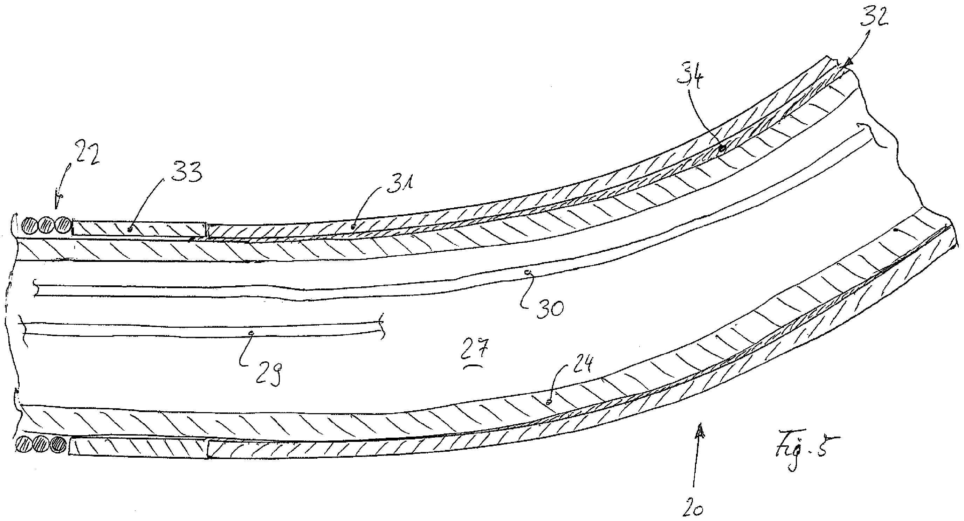

Wie Figur 4 und insbesondere Figur 5 zeigen, ist der Schlauch 24 zumindest abseits seines distalen Endes 12 von einem Mantel 31 umgeben, dessen Innendurchmesser den Außendurchmesser des Schlauchs 24 etwas übersteigt, sodass zwischen dem Schlauch 24 und dem Mantel 31 ein Spalt 32 festgelegt ist. Der Mantel 31 kann sich dabei von dem proximalen Ende 13 bis zu einer der Elektroden 21, 22 erstrecken. Optional kann zwischen dem distalen Ende des Mantels 31 und der Elektrode 22 ein Isolatorring 33 angeordnet sein, der sich an die Elektrode 22 anschließt.

Eine Besonderheit des erfindungsgenmäßen Ablationsinstruments 11 liegt in dem Krümmungsbereich 20, in welchem der Spalt 32 mit einem Kunststoff 34 gefüllt ist. Vorzugsweise erstreckt sich diese Kunststofffüllung jedoch nicht über die gesamte Länge des Ablationsinstruments 11, sondern ist im Wesentlichen auf den Krümmungsbereich 20 beschränkt. Es ist jedoch auch möglich, größere Abschnitte des Spalts 32 oder den gesamten Spalt 32 mit Kunststoff 34 aufzufüllen.

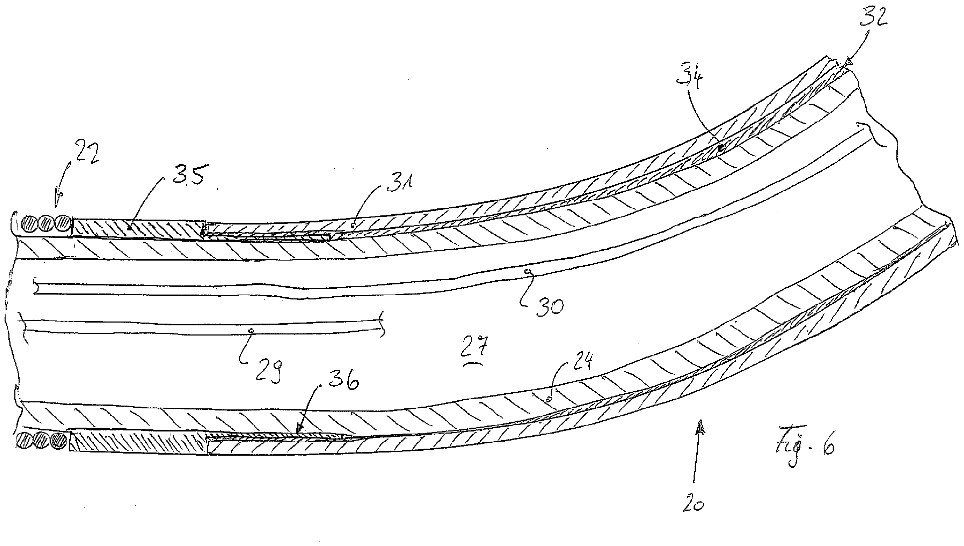

Anstelle des Isolatorrings 33 kann auch ein Ring 35 aus Metall oder Kunststoff vorgesehen sein, der insbesondere als Zentrierelement dient und aus Figur 6 hervorgeht. Der Ring 35 weist einen rohrförmigen Fortsatz 36 auf, der sich in den Spalt 32 erstreckt und den Mantel 31 relativ zu dem Schlauch 24 zentriert. Der den Spalt 32 im Krümmungsbereich 20 ausfüllende Kunststoff 34 kann sich zwischen den Mantel 31 und den Fortsatz 36 erstrecken und abdichten.

Der Kunststoff 34 ist vorzugsweise ein aushärtbarer Kunststoff, z.B. ein 2-Komponenten-Kunststoff, der bei der Herstellung des Instruments in flüssiger Form in den Spalt 32, insbesondere im Krümmungsbereich 20, eingebracht wird. Zur Ausbildung des Krümmungsbereichs 20 wird dieser kurz vor dem Einbringen des Kunststoffs oder kurz danach in eine gekrümmte Form überführt, wonach der Kunststoff 34 ausgehärtet wird. Dazu kann das Ablationsinstrument 11 in eine entsprechende Haltevorrichtung eingebracht werden, die dem Ablationsinstrument 11 im Krümmungsbereich 20 eine Bogenform aufzwingt. Nach dem Aushärten des Kunststoffs kann das Ablationsinstrument 11 dieser gebogenen Form entnommen werden, wobei derjenige Bereich des Ablationsinstruments 11, der beim Aushärten des Kunststoffs 34 in gebogener Form gehalten worden ist, den Krümmungsbereich 20 bildet. Die restlichen Abschnitte des Ablationsinstruments 11 liegen jedoch in gestreckter Form vor.

Das insoweit beschriebene Ablationsinstrument 11 arbeitet wie folgt:
Zum Einsatz am Patienten wird zunächst das Endoskop 10 in den Patienten, beispielsweise in das Bronchialsystem desselben, eingeführt und an der zu behandelnden Stelle platziert. Schon zuvor oder spätestens jetzt wird das Ablationsinstrument 11 über den Anschluss 18 durch den Arbeitskanal des Endoskops 10 an den Operationsort geschoben. Dabei wird das gemäß Figur 2 vorgebogene Ablationsinstrument 11 elastisch in seine gestreckte Form überführt und bis zu dem abgewinkelten Ende 16 des Schafts 15 vorgeschoben. Sobald der Krümmungsbereich 20 des Ablationsinstruments 11 das abgewinkelte distale Ende 16 des Schafts 15 erreicht, vollführt es, infolge seiner Bestrebung in die vorgekrümmte Form zurückzuschnappen, eine axiale Drehbewegung (zumindest sofern es nicht zufällig schon die richtige Drehorientierung um seine Achse A hat). Zugleich mit dem axialen Vorschieben des Ablationsinstruments 11 kann es mit seiner Sondenspitze, d.h. mit seinem Endstück 26 in zu behandelndes Gewebe eindringen bzw. eingestochen werden. Dazu kann gegebenenfalls auch das Endstück 26 mit Spannung und Strom versorgt werden, um das Einstechen in kompaktes Gewebe, beispielsweise Tumorgewebe, zu erleichtern oder kleinere Blutungen zu koagulieren. Die Stromzuführung zu dem Endstück 26 kann über das Zugmittel 29 oder auch über das Kapillarrohr 30 oder einen gesonderten Leiter erfolgen.

Aufgrund der durch den Krümmungsbereich 20 gegebenen Vorbiegung lässt sich das distale Ende 16 des Schafts 15 mit einem sehr engen Biegeradius von zum Beispiel weniger als 15 mm auch auf sehr große stumpfe Winkel gegenüber der Axialrichtung A von zum Beispiel über 145° abwinkeln, ohne dass ein Abknicken des Schlauchs 24 und somit ein Kollaps des Lumens 27 zu befürchten wäre. Zudem wirken der ausgehärtete Kleb- oder Kunststoff 34 sowie der Mantel 31 stützend und verhindern zusätzlich jede Abknicktendenz des Schlauchs 24. Infolgedessen kann das über das Kapillarrohr 30 herbeigeführte Kühlfluid auch in stark anguliertem Zustand des Ablationsinstruments 11 frei durch das Lumen 27 zurück zu dem Gerät 14 oder einem in der Nähe des proximalen Endes 13 vorgesehenen Auslass fließen, ohne zu stauen und somit ohne die Kühlwirkung an den Elektroden 21, 22 zu mindern oder ein Bersten hervorzurufen. Das erfindungsgemäße Instrument gestattet so auch die Behandlung von sonst schwer zugänglichen Tumoren oder sonstigen Gewebepartien, die nur über eine starke Abwinklung des Endes 16 des Endoskops 10 erreichbar sind.

Das erfindungsgemäße innengekühlte Ablationsinstrument 11 weist im Anschluss an sein distales Ende 12 einen Krümmungsbereich 20 auf, bei welchem das Ablationsinstrument 11 gegen seine Axialrichtung A abgewinkelt ist. Die Größe dieses Winkels ist vorzugsweise auf ein Drittel bis zwei Drittel, vorzugsweise die Hälfte, der maximalen Abwinklung festgelegt. Die maximale Abwinklung kann einen Betrag von 140° deutlich überschreiten. Durch die Vorkrümmung in dem Krümmungsbereich 20 wird ein Abknicken des Schlauchs des Ablationsinstruments und somit eine Beeinträchtigung der Elektrodenkühlung sowie das Auftreten eines hohen Staudruckes wirksam verhindert. Damit gestattet das erfindungsgemäße Ablationsinstrument 11 eine im Vergleich zu vollkommen gestreckten Ablationsinstrumenten freieres und noch stärker an medizinischen Aspekten und Gesichtspunkten ausgerichtetes Arbeiten. Die Behandlungssicherheit für den Patienten wird deutlich erhöht.

### Bezugszeichen:

- 10: Endoskop
- 11: Ablationssonde
- 12: distales Ende der Ablationssonde 11
- 13: proximales Ende der Ablationssonde 11
- 14: Gerät zum Betrieb der Ablationssonde 11
- 15: Schaft des Endoskops 10
- 16: distales Ende des Schafts 15
- 17: Bedienelement des Endoskops 10
- 18, 19: Anschlüsse des Endoskops 10
- A: Axialrichtung
- 20: Krümmungsbereich der Ablationssonde 11
- α: Winkel
- 21, 22: Elektroden
- 23: Isolator
- 24: Schlauch
- 25: Instrumentenspitze
- 26: Endstück
- 27: Lumen
- 28: Isolator
- 29: Zugmittel
- 30: Kapillarrohr
- 31: Mantel
- 32: Spalt zwischen Mantel 31 und Schlauch 24
- 33: Isolator
- 34: Kunststoff
- 35: Ring
- 36: Fortsatz

## Patentansprüche

1. Ablationsinstrument (11), insbesondere zur thermischen Abtötung eines Gewebevolumens durch Bestromung, mit einem flexiblen Schlauch (24), der wenigstens ein Lumen (27) aufweist, das sich von einem proximalen Ende (13) des Schlauchs (24) zu einem distalen Ende (12) des Schlauchs (24) erstreckt,
mit mindestens einer Elektrode (21, 22), die an dem distalen Ende (12) des Schlauchs (24) angeordnet ist, und
wobei das Ablationsinstrument (11) einen Krümmungsbereich (20) aufweist, in dem der Schlauch (24) ohne auf das Ablationsinstrument (11) einwirkende äußere Kräfte einem Bogen folgend ausgebildet ist.

2. Ablationsinstrument nach Anspruch 1, **dadurch gekennzeichnet, dass** das Lumen (27) an der Spitze des distalen Endes (12) geschlossen ist.

3. Ablationsinstrument nach Anspruch 2, **dadurch gekennzeichnet, dass** zum Verschluss des Schlauchs (24) ein Verschlussstück (26) vorgesehen ist, das mit einem sich von dem distalen Ende (12) in proximaler Richtung erstreckenden Zugmittel (29) verbunden ist.

4. Ablationsinstrument nach einem der vorstehenden Ansprüche, **dadurch gekennzeichnet, dass** in dem Schlauch (24) ein Kapillarkanal angeordnet ist, der eine oder mehrere Mündungen in der Nähe der mindestens einen Elektrode (21) aufweist.

5. Ablationsinstrument nach Anspruch 4, **dadurch gekennzeichnet, dass** der Kapillarkanal in einem sich durch das Lumen erstreckenden Kapillarrohr (30) ausgebildet ist.

6. Ablationsinstrument nach einem der vorstehenden Ansprüche, **dadurch gekennzeichnet, dass** die Elektrode (21) den Schlauch (24) umfassend ausgebildet ist wobei die Elektrode (21) durch einen den Schlauch (24) schraubenförmig umwindenden elektrischen Leiter gebildet ist.

7. Ablationsinstrument nach einem der vorstehenden Ansprüche, **dadurch gekennzeichnet, dass** die Elektrode (22) außerhalb des Krümmungsbereichs (22) angeordnet ist.

8. Ablationsinstrument nach einem der vorstehenden Ansprüche, **dadurch gekennzeichnet, dass** auf dem Schlauch (24) an seinem distalen Ende (12) in dem nicht gekrümmten Bereich desselben wenigstens eine weitere Elektrode (22) angeordnet ist.

9. Ablationsinstrument nach Anspruch 8, **dadurch gekennzeichnet, dass** beide Elektroden (21, 22) in einem Abstand zueinander, jedoch ansonsten gleich ausgebildet sind.

10. Ablationsinstrument nach einem der vorstehenden Ansprüche, **dadurch gekennzeichnet, dass** auf dem Schlauch (24) ein Mantel (31) angeordnet ist.

11. Ablationsinstrument nach Anspruch 10, **dadurch gekennzeichnet, dass** zwischen dem Mantel (31) und dem Schlauch (24) ein Spalt (32) ausgebildet ist.

12. Ablationsinstrument nach Anspruch 11, **dadurch gekennzeichnet, dass** in dem Spalt (32) wenigstens ein elektrischer Leiter angeordnet ist, der sich von der Elektrode (21) in proximaler Richtung erstreckt.

13. Ablationsinstrument nach einem der Ansprüche 10 oder 11, **dadurch gekennzeichnet, dass** der Spalt (32) wenigstens in dem Krümmungsbereich (20) mit einem Kunststoff (34) gefüllt ist.

14. Ablationsinstrument nach Anspruch 13, **dadurch gekennzeichnet, dass** der Kunststoff (34) ein flexibler Kunststoff ist.

15. Verfahren zur Herstellung eines Ablationsinstruments nach Anspruch 13, **dadurch gekennzeichnet, dass** der Kunststoff (34) ein aushärtbarer Kunststoff ist, der in den Spalt (32) eingebracht und das Ablationsinstrument (11) in dem Krümmungsbereich in eine Bogenform überführt und der Kunststoff (34) ausgehärtet wird.
